# EUROPEAN PATENT APPLICATION

(11) **EP 1 925 285 A1**
(43) Date of publication of application: **28.05.2008**
(21) Application number: 06024375.5
(22) Date of filing: 24.11.2006
(51) Int. Cl.: A61K 8/26, A61K 8/29, A61Q 5/12

(54) **Conditioner composition for keratin fibres**

(71) Applicant: KPSS-Kao Professional Salon Services GmbH, 64297 Darmstadt (DE)
(72) Inventor: Hofmann, Martin, 64673 Zwingenberg (DE); Tietjen, Ilka, 69207 Sandhausen (DE)

(57) **Abstract**

SUMMARY

The present invention relates to a leave -in conditioner composition in form of water in oil emulsion, particularly water in silicone oil emulsion for keratin fibres especially human hair. The present inventors have surprisingly found out a water in oil emulsion, in particular a water in silicone oil emulsion comprising volatile silicone oil, water in oil emulsifier and an internal water phase comprising synthetic mica coated with metal oxide or oxides and having a volume particle size distribution in the range of 1 to 750 µm conditions hair excellently and improves hair shine dramatically and gives hair smoothness and elasticity.

## Description

The present invention relates to a leave -in conditioner composition in form of water in oil emulsion, particularly water in silicone oil emulsion for keratin fibres especially human hair.

Conditioner either leave-in and rinse off types have been widely used in hair dressing area. In the last decade especially leave-in conditioning compositions have gained much attention starting from Europe and still increasingly growing in Asian markets.

Leavd-in conditioning compositions are especially found appropriate-by consumers as they can generally be applied at the sites where necessary and the processing time does not have to be predetermined and more importantly washing hair after certain processing time is not needed.

Shine is an important property of hair indicating its healthiness and cosmetically appealing look. Although shine enhancing preparations are widely available, there is still need for improvement.

The present inventors have surprisingly found out a water in oil emulsion, in particular a water in silicone oil emulsion comprising volatile silicone oil and an internal water phase comprising synthetic mica coated with metal oxide or oxides and having a volume particle size distribution in the range of 1 to 750 µm conditions hair excellently and improves hair shine dramatically and gives hair smoothness and elasticity.

Thus, the object of the present invention is a water in oil emulsion composition comprising at least one volatile silicone oil and at least one water in oil emulsifier and an internal water phase comprising synthetic mica coated with metal oxide or oxides and having a volume particle size distribution in the range of 1 to 750 µm.

With the term volume particle size distribution it is meant that synthetic mica coated with a metal oxide or oxides include particles with a size in the range of 1 to 750 µm measured as volume diameter.

Another object of the present invention is the use of a water in oil emulsion composition comprising at least one volatile silicone oil and at least one water in oil emulsifier and an internal water phase comprising synthetic mica coated with metal oxide or oxides and having a volume particle size distribution in the range of 1 to 750 µm for conditioning hair.

Compositions of the present invention are preferably leave in compositions. It should be noted that rinsing off after application should not be seen as totally excluded from the scope. The most beneficial effects of the compositions of the present invention are observed in leave in application, i.e. when the compositions are not rinsed off from hair after application.

Still another subject of the present invention is a process for conditioning wet and/or freshly cleansed hair wherein a water in oil emulsion composition comprising at least one volatile silicone oil and at least one water in oil emulsifier and an internal water phase comprising synthetic mica coated with metal oxide or oxides and having a volume particle size distribution in the range of 1 to 750 µm is applied to hair and without rinsing off, hair is dried.

Still further object of the present invention is a process for conditioning dry hair wherein a water in oil emulsion composition comprising at least one volatile silicone oil and at least one water in oil emulsifier and an internal water phase comprising synthetic mica coated with metal oxide or oxides and having a volume particle size distribution in the range of 1 to 750 µm is applied to hair.

The compositions of the present invention comprises at least one volatile silicone oil, As a rule all silicone oils commercially available defined as volatile are suitable for the present invention. Preferred are those of cyclo organopolysiloxanes with the general formula 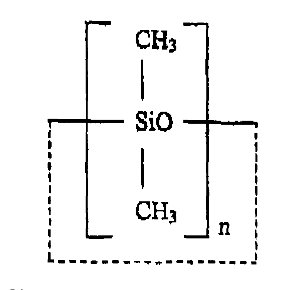 where n is a number between 3 and 7, which are available from Dow Corning with the trade name for example DC 245 which is cyclopentasiloxane where n=5.

Further volatile silicones suitable are known also from Dow Corning with the trade name DC 200 Fluid which includes a series of trisiloxanes with varying viscosities. Within the meaning of the present invention trisiloxanes with viscosity at 20°C below or equal to 50 mPa.s are suitable. More preferred are trisiloxanes with a viscosity below or equal to 20 mPa.s, most preferred are those of with a viscosity below or equal to 5 mPa.s.

Concentration of one or more volatile silicone oil is in the range of 10 to 50%, preferably 15 to 40% and more preferably 20 to 30% by weight, calculated to total composition.

Compositions of the present invention comprise one or more water in oil emulsifier. Suitable ones are known in the textbooks as water in oil emulsifiers with HLB values generally below 10. Examples are mono or diglycerides with a fatty acyl chain length of 16 to 22 C atoms and silicone surfactants, especially ethoxylated and/or propoxylated dimethicone with number of ethoxy and/or propoxy groups in the range of 5 to 25. Preferred are ethoxylated and propoxylated siliocne surfactants with number of ethoxy and propoxy groups each in the range of 10 to 20, more preferably 15 to 20. The most preferred emulsifiers are PEG/PPG 18/18 Dimethicone which is available from Dow Corning with the trade name DC 5225 C and PEG/PPG 20/15 Dimethicone available under trade name SF 1540 from GE silicones. Concentration of water in oil emulsifier is in the range of 0.1 to 5%, preferably 0.25 to 3%, more preferably 0.5 to 2.5% by weight, calculated to total composition.

Compositions of the present invention comprise in internal water phase synthetic mica coated with metal oxide or oxides having a volume particle size distribution in the range of 1 to 750 µm. Use of synthetic mica coated with metal oxide or oxides mainly in decorative cosmetics is disclosed in an international patent application of Sun Chemical Corporation published with a number WO 2005/065632 A1. In the document synthetic mica and coated synthetic mica with at least one metal oxide or oxides is disclosed in detail. The content of the document is included herewith by reference.

Suitable metal oxide or oxides for coating synthetic mica are titanium dioxide, chromium oxide, ferric oxide or mixtures thereof. In the present invention the preferred is synthetic mica coated with titanium dioxide. Such materials are commercially available from Sun Chemical Corporation and known with their INCI names Synthetic Fluorphologopite.

The volume particle size distribution of synthetic mica coated with a metal oxide or oxides is in the range of 1 to 750 µm, preferably 1 to 250 µm, more preferably 1 to 100 µm and most preferably 20 to 95 µm. The particle sizes referred are relating to the volume particle size distribution meaning that particles found in the coated synthetic mica having volume particle size in the given ranges.

Concentration of synthetic mica coated with at least metal oxide or oxides is from 0.001 to 10%, preferably 0.05 to 7.5%, more preferably 0.1 to 5% and most preferably 0.25 to 2.5% by weight calculated to total composition.

Compositions of the present invention comprises preferably an inorganic salt such as sodium chloride, magnesium sulphate, magnesium chloride at a concentration of 0.1 to 3%, preferably 0.25 to 2% and more preferably 0.5 to 1.5% by weight calculated to total composition. Preferred are sodium chloride and magnesium sulphate.

Composition of the present invention can comprise one or more non-volatile oil(s) in the oil phase. Suitable ones are non-volatile silicones such as dimethicone or dimethiconol commercially available for example from Dow Coming, or arylated non volatile silicones such as phenyl trimethicone also available from Dow corning. Further suitable oils are natural oils such as olive oil, almond oil, avocado oil, ricinus oil, jojba oil, coconut oil, palm oil, sesame oil, peanut oil, whale oil, sunflower oil, peach kernel oil, wheat germ oil, macadamia nut oil, night primrose oil, castor oil, or soya oil, lanolin and the derivatives thereof or their mixture, and mineral oil such as paraffin oil or petrolatum.

Further, compositions of the present invention can comprise fatty acid esters as oily substances such as isopropyl myristate, palmitate, stearate and isostearate, oleyl oleate, isocetyl stearate, hexyl laurate, dibutyl adipate, dioctyl adipate, myristyl myristate, oleyl erucate, cetyl palmitate, etc.

The above mentioned non-volatile oils can also be used in mixture with each other. Nonvolatile oils may be added into the compositions of the present invention at a concentration in the range of 0.01 to 5%, preferably 0.05 to 3%, more preferably 0.1 to 2% by weight, calculated to total composition.

Compositions of the present invention can comprise one or more fixing polymer(s) selected from anionic, cationic, amphoteric and nonionic ones or their mixtures. Although not limiting, one of the points in selecting fixing polymer is that the polymer should be soluble in the rest of the composition, preferably in water phase, without leaving undissolved rests.

Suitable anionic polymers are vinyl alkyl ether, in particular methyl vinyl ether/maleic acid copolymers, obtained by hydrolysis of vinyl ether/maleic anhydride copolymers, distributed under the trade name "Gantrez® AN or ES". These polymers may also be partly esterified, as for example, "Gantrez® ES 225" or "ES 435", the ethyl ester of an ethyl vinyl ether/maleic acid copolymer, or the butyl or isobutyl ester thereof.

Further useful anionic polymers are in particular vinyl acetate/crotonic acid or vinyl acetate/vinyl neodecanoate/crotonic acid copolymers of the type "Resyn®"; sodium acrylate/vinyl alcohol copolymers of the type "Hydagen® F", sodium polystyrene sulfonate, e.g.. "Flexan® 130"; ethyl acrylate/acrylic acid/N-tert.-butyl acrylamide copolymers of the type "Ultrahold®"; vinyl pyrrolidone/vinyl acetate/itaconic acid copolymers, acrylic acid/acrylamide copolymers or the sodium salts thereof.

Suitable non-ionic polymers are polyvinyl pyrrolidone, polyvinylpyrrolidone/vinylacetate copolymer and polyvinylpyrrolidone/vinylacetate/vinylpropionate copolymer. Preferred is polyvinylpyrrolidone/vinylacetate copolymer, In addition to these fixing polymers derivatives of natural polymers such as hydroxyl ethylcellulose may also be used alone or in combination with one of the fixing polymers mentioned above.

Suitable cationic polymers include Polyquaternium-4, Polyquaternium-11, Polyquaternium-16, Polyquaternium-18, Polyquatemium-24, Polyquaternium-28, Polyquaternium-46, polyvinylpyrrolidone/dimethylaminoethyl methacrylate, and cationically derivatized natural polymers. Preferred are Polyquaternium-4, Poiyquaternium-11 and Polyquaternium-16.

Suitable amphoteric polymers are those available from the company National Starch under the trade name Amphomer such as Octylacrylamide/Acrylates/Butylaminoethyl methacrylate copolymer, those available under the trade name Diaformer (methacryloylethylbetaine/methacrylates copolymer). Preferred are the ones available under the trade name Amphomer.

As a fixing polymer silicone containing polymers either grafted or block copolymer are also useful. Suitable and the preferred one is Polysilicone-9.

Concentration of any of the fixing polymer or their mixtures is in the range of 0.1 to 15%, preferably 0.5 to 10%, more preferably 1 to 7.5% by weight calculated to total composition.

Compositions may furthermore comprise other conditioning ingredients. As a rule water soluble or miscible conditioning ingredients should be added to the water phase and oil soluble or miscible ones should be present in oil phase although partitioning between the two phases during production and/or storage may occur. Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₁ CO (OCH₂CH₂)ₙ OH

R₁ CO (OCH₂CH₂)ₙ O OC R₂

where R₁ and R₂ are independent from each other saturated, unsaturated or branched or non-branched alkyl chain with 7 to 21 C atoms and n is typically 2 - 100. Typical concentration range for any of the additional conditioners mentioned above other than cationic conditioning compounds can be in the range of 0,01 to 10% by weight, preferably 0,05 - 7.5% by weight, more preferably 0.1-5% by weight calculated to the total composition.

Composition of the present invention can comprises cationic polymers as conditioning agents, Those are cationic cellulose type polymers know as Polymer JR type from Amerchol such as Polyquaternium 10 or cationic guar gum known with trade name Jaguar from Rhane-Poulenc and chemically for example Guar hydroxypropyl trimonium chloride, Furthermore, chitosan and chitin can also be included in the compositions as cationic natural polymers.

Furthermore, it has especially been found suitable those cationic polymers known with their CTFA category name Polyquaternium. Typical examples of those Polyquaternium 6, Polyquaternium 7, Polyquaternium 10, Polyquaternium 22, Polyquaternium 30, Polyquaternium 37, Polyquaternium 36, and Polyquaternium 67.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quatemium-53, Quaternium-60, Quaternium-61, Quaternium-72, Quaternium-78, Quaternium-80, Quaternium-81, Quaternium-81, Quaternium-82, Quaternium-83 and Quaternium-84,

The compositions according to the present invention can also comprise further agents, such as protein hydrolyzates and polypeptides, e.g. keratin hydrolyzates, collagen hydrolyzates of the type "Nutrilan" or elastin hydrolyzates, as well as, in particular vegetable, optionally cationized protein hydrolyzates, for example "Gluadin".

Additionally, natural plant extracts can as well form part of the compositions of the present invention. Those are incorporated usually in an amount of about 0.01 % to about 5%, preferably 0,05 % to 3 %, in particular,0.1 % to 2 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, green tea, blue lotus flower, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapone" products and "Herbasol^{R} ". Extracts and the preparation thereof are also described in "Hagers Handbuch der pharmazeutischen Praxis", 4th Ed.

The compositions may contain one or more organic solvent(s) such as ethanol, propanol, isopropanol, benzyl alcohol, benzyloxyethanol, alkylene carbonates such as ethylene carbonate and propylene carbonate, phenoxyethanol, butanol, isobutanol, cyclohexane, cyclohexanol, hexyleneglycol, ethylenecarbonate, ethyleneglycol monoethylether, ethylene glycol monobutyl ether, ethylene glycol monophenyl ether, 1-phenylethylalcohol, 2-phenylethylalcohol, o-methoxyphenol. Concentration of organic solvents in the composition should not exceed 10% by weight, preferably within the range of 0.1 to 5% by weight calculated to total composition. It should be noted that that presence of organic solvent may effect the stability of emulsion and therefore type and concentration should be selected carefully.

Compositions of the present invention can comprise one or more UV filter(s) either for stabilization of the product colour or for protection of hair from environmental influences such as loss of elasticity, loss of hair colour (bleaching effect of sun light), The UV-absorbing substance is preferably selected from the following compounds: Polysilicone-15, 4-Aminobenzoic acid and the esters and salts thereof, 2-phenyl benzimidazole-5-sulfonic acid and the alkali and amine salts thereof, 4-dimethyl aminobenzoic acid and the.esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxybenzophenone, 2-hydroxy-4-methoxybenzophenone and its 5-sulfonic acid or the sodium salt thereof, 2.2'-dihydraxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzylidenecampher, 3-(4'-sulfo)-benzyl-idenebomane-2-one and the salts thereof and/or 3-(4'-methyl benzylidene)-DL-campher, polysilicone-15. The preferred amount of the UV-absorber ranges from about 0.01 % to 2.5%, more preferably from 0.05 % to 1 % by weight, calculated to the total composition. Water soluble UV filters should be added into the water phase and oil soluble ones should be part of the oil phase. In the preferred embodiment of the present invention the compositions comprise at least one water soluble UV filter and at least one oil soluble UV filter at above given concentration ranges as the total UV filter content.

The compositions of the present invention can comprise one or more hair-restructuring agents. The hair restructuring agents preferred are especially the ones disclosed in the German patent DE 197 51 550 C2. Namely they are ceramide type of compounds, fatty acids and phytosterol or their mixtures.

Preferred ceramide compound is cetyl-PG-hydroxyethylpalmitamide.

Preferred fatty acids are those with 10 to 24 carbon atoms and especially with 16 to 22 carbon atoms.

Sterols,especially the phytosterols, are as well preferred hair restructuring agents as disclosed in the above mentioned german patent. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

The concentration of ceramide in the compositions of the present invention can be in the range of 0.01 to 2% and especially 0.05 to 1% by weight calculated to the total composition, The fatty acids may be contained at a level of 0,01 to 2.5% and especially 0,05 to 1% by weight calculated to the total composition. Phytosterol concentration is less than 1% and preferably in the range of 0.01 to 0.5% by weight calculated to the total composition. It should be noted without limiting the use of those ingredients the effect of those hair restructuring ingredients is especially elevated when used in combination with penetration enhancers.

Further conditioning ingredients may be added to the water phase of the compositions of the present invention are cationic surfactants of the following formula 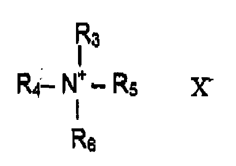 where R₃ is a saturated or unsaturated, branched or non-branched alkyl chain with 8-22 C atoms or

R₇CO NH (CH₂)ₙ

where R₇ is saturated or unsaturated, branched or non-branched alkyl chain with 7-21 C atoms and n has typical value of 0 - 4 or

R₈CO O (CH₂)ₙ

where R₈ is saturated or unsaturated, branched or non-branched alkyl chain with 7-21 C atoms and n has typical value of 0 - 4, and
R₄ is a hydrogen, saturated or unsaturated, branched or non-branched alkyl chain with 1-22 C atoms or

R₇CO NH (CH₂)ₙ

where R₇ is saturated or unsaturated, branched or non-branched alkyl chain with 7-21 C atoms and n has typical value of 0 - 4 or

R₈CO O (CH₂)ₙ

where R₈ is saturated or unsaturated, branched or non-branched alkyl chain with 7-21 C atoms and n has typical value of 0 - 4,
and R₅ and R₆ are independent from each other H or lower alkyl chain with 1 to 4 carbon atoms or ethoxy or propoxy group with number of ethoxy or propoxy groups varying in the range of 0 to 4, and X is chloride, bromide or methosulfate.

With the term cationic surfactant it is meant that the surfactant carries a cationic charge when used in the compositions, In other words, compounds having no cationic charge but when added into the compositions protonate and therewith become cationic are also included within the definition of cationic surfactant. Example to such may be stearyldimethylamine and PEG-2 Cocamine which are as a compound-not carrying a cationic charge but when used in a composition having acidic pH becomes cationic by protonation.

Suitable cationic surfactants as conditioning agents are, for example, long-chain quaternary ammonium compounds which can be used alone or in admixture with one another, such as cetyl trimethyl ammonium chloride, myristoyl trimethyl ammonium chloride, behentrimonium chloride, trimethyl cetyl ammonium bromide, stearyl trimethyl ammonium chloride, dimethyl stearyl ammonium chloride, dimethyl dihydrogenated tallow ammonium chloride, stear trimonium chloride, stearamidopropyldimethylamoonium chloride, dipalmitoyl dimonium chloride, distearyl dimethyl ammonium chloride, stearamidopropyl trimonuim chloride.

Further examples to the cationic surfactants are so called esterquats available on the market, for example, under the trade names "Schercoquat^{®}", "Dehyquart^{®} L80" and "Tetranyl^{®}". Still further examples are so called amidoquatsAgain available on the market, for example, under the trade name "INCROQUAT^{à} HO" or "OCS".

Concentration of cationic surfactants may be in the range of 0.01 to 2%, preferably 0.05 to 1.5%, more preferably 0.1 to 1% by weight calculated to total composition. Any incompatibility with other used substances should be taken into account.

Additionally compositions of the present invention can comprise all substances customarily found In cosmetic compositions such as fragrance, preservative, complexing agents, dyestuffs for colouring composition etc.

The following examples are to illustrate the invention but not to limit.

### Example 1

| | % by weight |
|---|---|
| Cyclopentasiloxane | 25 |
| PEG/PPG - 18/18 Dimethicone* | 1.4 |
| Synthetic fluorphologopite** | 0.5 |
| Sodium chloride | 1.1 |
| Fragrance, preservative, dyestuff | q.s |
| Water | q.s. to 100 |

| | |
|---|---|
| *: used as a raw material DC 5225 C and therefore minor amounts of other cyclosiloxanes are also present. **: Synthetic fluorphologopite used is commercially available from Sun Chemical Corporation under the trade name SunShine Glitter White with a particle size distribution in the range of 20 to 95 µm. | |

Above composition was prepared by dispersing PEG/PPG - 18/18 Dimethicone in cyclopentasiloxanes which was combined with water containing dispersed Synthetic fluorphologopite and dissolved sodium chloride, dyestuff and preservative under agitation and subsequently homogenised with Ultrathorax at 9000 rpm.

The above composition used on dry and wet hair either wetted or cleansed and towel dried and it was observed that hair gained excellent shine, smoothness and elasticity. Furthermore, it was found that spreading on hair was very easy.

### Example 2

| | % by weight |
|---|---|
| Cyclopentasiloxane | 15 |
| Trisiloxane * | 10 |
| PEG/PPG - 20/15 Dimethicone** | 1.4 |
| Synthetic fluorphologopite*** | 0.5 |
| Magnesium chloride | 1.0 |
| Fragrance, preservative | q.s |
| Water | q.s. to 100 |

| | |
|---|---|
| *: DC 200 Fluid with a Viscosity of 1 cst **: used as a raw material SF 1540 ***: Synthetic fluorphologopite used is commercially available from Sun Chemical Corporation under the trade name SunShine Glitter White with a particle size distribution in the range of 20 to 95 µm. | |

The composition was prepared in a similar way as in example 1.

Similar effects were observed when used on dry and wet hair as in example 1.

### Example 3

| | % by weight |
|---|---|
| Cyclopentasiloxane | 15 |
| Trisiloxane * | 10 |
| PEG/PPG -18/18 Dimethicone** | 1.4 |
| Synthetic fluorphologopite*** | 0,4 |
| VPNA copolymer | 1.5 |
| Magnesium sulphate | 0.9 |
| Fragrance, preservative, dyestuff | q.s |
| Water | q.s. to 100 |

| | |
|---|---|
| *: DC 200 Fluid with a viscosity of 1 cst **: used as a raw material DC 5225 C and therefore minor amounts of other cyclosiloxanes are also present. ***: Synthetic fluorphologopite used is commercially available from Sun Chemical Corporation under the trade name SunShine Glitter White with a particle size distribution in the range of 20 to 95 µm. | |

The composition was prepared in a similar was as in example 1. The VP/VA polymer was dissolved in water.

Similar effects were observed when used on dry and wet hair as in Example 1. Additionally hair treated with the above composition had excellent body and volume.

### Example 4

| | % by weight |
|---|---|
| Trisiloxane * | 24.5 |
| Isopropyl palmitate | 0.5 |
| Octylmethoxycinnamate | 0.2 |
| PEG/PPG - 18/18 Dimethicone** | 1.4 |
| Synthetic fluorphologopite*** | 0.4 |
| Polyquaternium 11 | 1.5 |
| Magnesium sulphate | 0.9 |
| Fragrance, preservative, dyestuff | q.s |
| Water | q.s. to 100 |

| | |
|---|---|
| *: DC 200 Fluid with a viscosity of 1 cst **: used as a raw material DC 5225 C and therefore minor amounts of other cyclosiloxanes are also present. ***: Synthetic fluorphologopite used is commercially available from Sun Chemical Corporation under the trade name SunShine Glitter White with a particle size distribution in the range of 20 to 95 µm. | |

The composition was prepared in a similar was as in example 1. The Polyqauternium-11 was dissolved in water and isopropyl palmitate and W filter was added into oil phase.

Similar effects were observed when used on dry and wet hair as in Example 3.

### Example 5

| | % by weight |
|---|---|
| Trisiloxane * | 24.5 |
| Isopropyl stearate | 0.3 |
| Polysilicone-15 | 0.2 |
| Polysllicone-9 | 0.5 |
| PEG/PPG - 18/18 Dimethicone** | 1.5 |
| Synthetic fluorphologopite*** | 0.6 |
| Polyquaternium 11 | 1.5 |
| Ethanol | 3.0 |
| Sodium chloride | 1.2 |
| Fragrance, preservative, dyestuff | q.s |
| Water | q.s, to 100 |

| | |
|---|---|
| *: DC 200 Fluid with a viscosity of 1 cst **: used as a raw material DC 5225 C and therefore minor amounts of other cyclosiloxanes are also present. ***: Synthetic fluorphologopite used is commercially available from Sun Chemical Corporation under the trade name SunShine Glitter White with a particle size distribution in the range of 20 to 95 µm. | |

The above composition was prepared in a similar way as Example 1. Polysilicone 9 is added as 30% by weight ethanolic solution into water phase. UV filter polysilicone 15 was added into oil phase.

### Example 6

| | % by weight |
|---|---|
| Trisiloxane * | 15.5 |
| Cyclopentasiloxane | 11.0 |
| Phenyltrimethicone | 0.3 |
| Polysilicone-15 | 0.2 |
| PEG/PPG - 18/18 Dimethicone** | 1.5 |
| Synthetic fluorphologopite*** | 0.6 |
| Acrylates/butylacrylamide copolymer | 1.5 |
| Ethanol | 3.0 |
| Benzophenone-4 | 0.3 |
| Sodium chloride | 0.9 |
| Fragrance, preservative, dyestuff | q.s |
| Water | q.s. to 100 |

| | |
|---|---|
| *: DC 200 Fluid with a viscosity of 1 cst **: used as a raw material DC 5225 C and therefore minor amounts of other cyclosiloxanes are also present. ***: Synthetic fluorphologopite used is commercially available from Sun Chemical Corporation under the trade name SunShine Glitter White with a particle size distribution in the range of 20 to 95 µm. | |

### Example 7

| | % by weight |
|---|---|
| Trisiloxane * | 15.5 |
| Cyclopentasiloxane | 11.0 |
| Phenyltrimethicone | 0.3 |
| Polysilicone-15 | 0,2 |
| PEG/PPG - 20/15 Dimethicone** | 1.5 |
| Synthetic fluorphologopite*** | 0.6 |
| Ethanol | 3.0 |
| Cetrimonium chloride | 0.5 |
| Sodium chloride | 0.9 |
| Fragrance, preservative, dyestuff | q.s |
| Water | q.s. to 100 |

| | |
|---|---|
| *: DC 200 Fluid with a viscosity of 1 cst **: used as a raw material SF 1540. ***: Synthetic fluorphologopite used is commercially available from Sun Chemical Corporation under the trade name SunShine Glitter White with a particle size distribution in the range of 20 to 95 µm. | |

## Claims

1. Composition for keratin fibres especially for hair in form of a water in oil emulsion **characterised in that** it comprises at least one volatile silicone oil and at least one water in oil emulsifier and an internal water phase comprising synthetic mica coated with metal oxide or oxides and having a volume particle size distribution in the range of 1 to 750 µm.

2. Composition according to claim 1 **characterised in that** it comprises as a volatile silicone cyclomethicone and/or dimethicone with a viscosity below or equal to 50 mPa.s measured at 20°C.

3. Composition according to claims 1 and 2 **characterised in that** it comprises volatile silicone at a concentration of 10 to 50% by weight calculated to total composition.

4. Composition according to claims 1 to 3 **characterised in that** it comprises as water in oil emulsifier a silicone surfactant with 5 to 25 ethoxy and/or propoxy groups.

5. Composition according to claim 4 **characterised in that** water in oil emulsifier is selected from PEG/PPG 18/18 Dimethicone and PEG/PPG 20/15 Dimethicone and their mixtures.

6. Composition according to any of the preceding claims **characterised in that** it comprises water in oil emulsifier at a concentration of 0.1 to 5% by weight calculated to total composition.

7. Composition according to any of the preceding claims **characterised in that** it comprises synthetic mica coated with metal oxide or oxides having a volume particle size distribution in the range of 1 to 750 µm at a concentration of 0.001 to 10% by weight calculated to total composition.

8. Composition according to any of the preceding claims **characterised in that** it comprises one or more non-volatile oil(s) at a concentration of 0.01 to 5% by weight calculated to total composition.

9. Composition according to any of the preceding claims **characterised in that** it comprises one or more fixing polymer(s) selected from anionic, cationic, non-ionic and cationic or their mixtures at a concentration of 0.1 to 15% by weight calculated to total composition.

10. Composition according to any of the preceding claims **characterised in that** it comprises one or more hair conditioning compound(s).

11. Composition according to any of the preceding claims **characterised in that** it comprises one or more organic solvent(s).

12. Composition according to any of the preceding claims **characterised in that** it comprises one or more UV filter(s).

13. Composition according to any of the preceding claims **characterised in that** it comprises an inorganic salt.

14. Composition according to any of the preceding claims **characterised in that** it is a leave in hair conditioning composition.

15. Use of a composition according to any of the preceding claims for conditioning keratin fibres especially human hair,

16. Process for conditioning hair either wet and/or freshly cleansed hair or dry hair wherein a composition according to claims 1 to 13 is applied onto hair and without rinsing off, hair is dried or left to dry.
